# EUROPÄISCHE PATENTSCHRIFT

(11) **EP 0 697 174 B1**
(45) Veröffentlichungstag und Bekanntmachung des Hinweises auf die Patenterteilung: **15.03.2000**
(21) Anmeldenummer: 94112805.0
(22) Anmeldetag: 17.08.1994
(51) Int. Cl.: A01N 59/00, A61L 2/00

(54) **Verfahren zur Herstellung antimikrobieller Wirkstoffe**
Process for the preparation of antimicrobial agents
Procédé de préparation d'agents antimicrobiens

(43) Veröffentlichungstag der Anmeldung: 21.02.1996
(73) Patentinhaber: HEITLAND UND PETRE INTERNATIONAL GmbH, D-29229 Celle (DE)
(72) Erfinder: Ohme, Roland, Dr. rer.nat, 12589 Berlin (DE); Ballschuh, Detlef, Dr. rer.nat., 12524 Berlin (DE); Jülich, Wolf-Dieter, PD Dr.rer.nat.habil., 17489Greifswald (DE); Kramer, Axel, Prof. Dr. med., 17489 Greifswald (DE)
(74) Vertreter: Wehser, Wulf, Dipl.-Ing.

(56) Entgegenhaltungen:
- DE-A- 687 217
- DE-A- 1 169 901
- DE-A- 3 434 468
- DE-A- 4 137 544
- DE-A- 4 200 140
- US-A- 4 837 008
- US-A- 4 988 500

## Beschreibung

Die Erfindung betrifft ein Verfahren zur Herstellung antimikrobieller Verbindungen auf Basis von Percabamid-Einschlußverbindungen. In der DE 42 00 140 A1 werden Einschlußverbindungen, die aus Harnstoff, H₂O₂ und Tensiden bestehen Beschrieben, wobei die Einschlußverbindungen durch Kristallisation der gemeinsamen wäßrigen Lösung der Komponenten hergestellt werden.

DE 34 34 468 A1 beschreibt antiseptische Produkte, die durch Besprühen oder trockenes Vermischen eines H₂O₂-Harnstoff-Adduktes mit Polyethylenglykol erhältlich sind.

Die DE-AS 1 169 901 beschreibt ein Verfahren zur Herstellung von Wasserstoffperoxid enthaltenden Pasten, wobei das Wasserstoffperoxid und/oder Percarbonat in Polyethylenoxiden gelöst werden.

Nachteilig, insbesondere bei dem Verfahren der erstgenannten Druckschrift ist es, daß der H₂O₂-Gehalt nicht an spezielle antimikrobielle Anwendungen angepaßt werden kann, weil das Kristallwachstumsverhalten aus der wäßrigen Lösung nur bestimmte stoffabhängige Konzentrationen zuläßt. Der Aufbau von Mehrkomponenten-Einschlußverbindungen unter Einbeziehung weiterer Wirkstoffe in das System Harnstoff/Tensid/H₂O₂ ist mithin bei der Kristallisation aus wäßriger Lösung nicht möglich.

Der Erfindung liegt demgemäß die Aufgabe zugrunde, ein Verfahren der eingangs genannten Art zu schaffen, mit welchem es möglich ist, neue antimikrobielle Wirkstoffe mit einem breiten Wirkungsspektrum unter Einbeziehung von physiologisch tolerierbaren Tensiden auf naturnaher Basis herzustellen.

Diese Aufgabe wird erfindungsgemäß dadurch gelöst, daß man die Percarbamid-Einschlußverbindung und ein physiologisch tolerierbares Tensid direkt miteinander bei 50° bis 130°C zu einer Mehrkomponenten-Einschlußverbindung umsetzt.

Mit diesem Verfahren wird erreicht, daß sich haut-, schleimhaut- und gewebeverträgliche Einschlußverbindungen des Harnstoffs mit H₂O₂ und Tensiden herstellen lassen, die sich in überraschender Weise für die Konservierung von kosmetischen und pharmazeutischen Zubereitungen besonders gut eignen und außerdem aufgrund ihres breiten Wirkungsspektrums für den Schutz technischer Produkte sowie Spezialaufgaben der Desinfektion und Sterilisation einsetzen lassen. Neben der erheblichen Erweiterung des Anwendungsbereiches der mit dem erfindungsgemäßen Herstellungsverfahren erzeugten Stoffe wird es außerdem möglich, weitere Mehrkomponenten-Einschlußverbindungen mit zusätzlichen Wirkstoffen zu erzeugen, so daß der mit dem erfindungsgemäßen Herstellungsverfahren erzeugte Stoff auf dem hier zur Rede stehenden Gebiet nahezu universell einsetzbar ist.

Besonders bevorzugt wird das Verfahren bei etwa 100°C bis etwa 130°C durchgeführt.

Die genannten vergleichsweise hohen Temperaturen fördern die Entstehung der Einschlußverbindungen beziehungsweise machen sie erst möglich.

Die Herstellung des Wirkstoffes wird weiter gefördert und beschleunigt, wenn die Erwärmung unter gleichzeitiger ständiger Bewegung zum Zwecke der Durchmischung (Rühren) der Einzelkomponenten erfolgt. Wie oben dargelegt, wird die Breite des Wirkungsspektrums der mit dem erfindungsgemäßen Verfahren hergestellten Wirkstoffe durch die Erwärmung und Wasserfreiheit möglich, wobei es besonders zweckmäßig ist, wenn die Grundlage des Stoffes durch eine von Haus aus wasserfreie Einschlußverbindung gebildet ist.

Die Einschlußverbindung besteht aus einer Harnstoff-Wasserstoffperoxid (Percarbamid)Verbindung. Es wird hier davon Gebrauch gemacht, daß bei chemischen Umsetzungen die an sich bekannte Einschlußverbindung Wasserstoffperoxid in Harnstoff (Percarbamid) ohne Anwesenheit von Tensiden dem reinen Wasserstoffperoxid überlegen ist.

In besonders vorteilhafter Weise wird die Einschlußverbindung mit den Tensiden innig durchmischt. Die Mischung kann gegebenenfalls mit weiterem Harnstoff innig gemischt werden und durch kurzes Erwärmen im Temperaturbereich von 50°C bis 130°C durch einen Sinterungs- beziehungsweise Schmelzprozeß in Drei- oder Mehrkomponenten-Einschlußverbindung umgewandelt werden. Die Temperatur wird durch die Empfindlichkeit der Komponenten einerseits und durch das Schmelzverhalten des Mehrkomponentensystems andererseits bestimmt.

Die Tenside können in an sich bekannter Weise anionischer, zwitterionischer, kationischer, nichtionischer oder polyfunktioneller Art sein.

Erfindungsgemäß wird in manchen Fällen ein hautfreundlicher oder hautpflegender Wirkstoff zugefügt, beispielsweise Allantoin, Panthenol etc. Damit werden einerseits das Wirkungsspektrum und die Einsatzmöglichkeiten der Einschlußverbindungen erweitert, andererseits wird der Schmelzpunkt des Systems herabgesetzt, was verfahrenstechnische Vorteile bringt. Durch niedrigere Umwandlungstemperaturen und/oder kürzere Verweilzeiten wird der Einsatz auch empfindlicherer Komponenten möglich.

Zur Herstellung von Mehrkomponenten-Einschlußverbindungen mit H₂O₂, Tensid, einem weiteren Wirkstoff und Harnstoff als Komponenten kann man erfindungsgemäß auch so verfahren, daß Percarbamid mit einer vorgefertigten Zweikomponenten-Einschlußverbindung aus Tensid und Harnstoff, gegebenenfalls weiterem Harnstoff und gegebenenfalls einer weiteren Komponente gemischt und umgesetzt werden. Man kann ferner so verfahren, daß der Wirkstoff als Harnstoff-Einschlußverbindung zugesetzt wird. Auch für die Tensidkomponenten besteht die Möglichkeit, freies Tensid oder eine vorgefertigte Harnstoff-Einschlußverbindung zuzusetzen. Es entspricht auch dem erfindungsgemäßen Verfahren, zunächst eine Mehrkomponenten-Einschlußverbindung aus Harnstoff, einem oder mehreren Wirkstoffen und einem oder mehreren Tensiden vorzufertigen, welches anschließend mit Percarbamid in vorwählbaren Mischungsverhältnissen gemischt und durch Erwärmen umgewandelt und homogenisiert wird. Die Arbeitsweise mit Tensid-Harnstoff-Einschlußverbindungen wird dann den Vorzug bekommen, wenn das Tensid nur als Paste oder flüssig oder in einer schwer zu reinigenden Form gewonnen wird. Durch die Vorfertigung einer Harnstoff-Einschlußverbindung kann erfindungsgemäß eine reinere, besser lagerfähige und besser dosierbare Form des Tensids zur Verfügung gestellt werden.

Die erfindungsgemäßen antimikrobiellen Wirkstoffe finden Anwendung zur Erzielung von besonders haut-, schleimhaut- und gewebeverträglichen Endprodukten. Sie sind demzufolge besonders für die Konservierung von pharmazeutischen und kosmetischen Zubereitungen geeignet. Durch das erfindungsgemäße Herstellungsverfahren kann der Wirkstoffgehalt und das Mischungsverhältnis der Komponenten an die für den jeweiligen Verwendungszweck notwendigen Bedingungen angepaßt werden. Dadurch sind die erfindungsgemäßen Wirkstoffe auch für die Konservierung von technischen Produkten sowie für Spezialaufgaben der Desinfektion und Sterilisation einsetzbar. In Kombination mit Gammastrahlung eignen sie sich zur Sterilisation besonders empfindlicher Materialien, zum Beispiel von enzymatischen Biosensoren. Das erfindungsgemäße Verfahren gestattete die Verwendung besonders naturnaher und naturidentischer Tenside. Dadurch ist die Akzeptanz der erfindungsgemäßen Einschlußverbindungen gegenüber anderen antimikrobiellen aber körperfremden und unphysiologischen Stoffen wesentlich größer.

Das erfindungsgemäße Verfahren ist mithin besonders zur antimikrobiellen Behandlung, zum Beispiel zur Erzielung von Sporenfreiheit, geeignet, wobei die Einschlußverbindung zur Behandlung von empfindlichen Materialien, wie Biosensoren, verwendet werden kann. Eine Sterilisierung kann ferner unter zusätzlicher Verwendung von Gammastrahlen herbeigeführt werden.

Die Erfindung wird im folgenden anhand der nachstehend aufgeführten Beispiele näher erläutert.

### Beispiel 1

### Herstellung der Wasserstoffperoxid-Harnstoff-Einschlußverbindung:

100g 33%iges H₂O₂ wird im Wasserbad auf 30° bis 40°C erwärmt. Unter Rühren trägt man 100g Harnstoff allmählich ein und läßt unter Kühlen zur Kristallisation stehen. Es kristallisiert ein Percarbamid mit 30 % H₂O₂-Gehalt aus, das für die nachfolgenden Beispiele als Ausgangsmaterial eingesetzt wird.

### Beispiel 2

### Herstellung einer 2-Komponenten-Einschlußverbindung eines naturnahen Tensids in Harnstoff:

2g Hexadecansäuresorbitanester werden unter Rühren mit 18g Harnstoff bei 100° bis 125°C miteinander verschmolzen. Nach Entstehen eines homogenen Gemisches läßt man unter Rühren abkühlen und erhält eine feste Einschlußverbindung mit einem Tensidgehalt von 10 %.

### Beispiel 3

2g eines Tensidgemisches von 66 % Tetradecansäuremonoglycerid mit 34 % Hexadecansäurediethanolamid werden, wie in Beispiel 2 beschrieben, zu einer Tensid-Einschlußverbindung mit 10 % Tensidgehalt umgesetzt.

### Beispiel 4

5g des nach Beispiel 2 hergestellten Produktes werden mit 5g des in Beispiel 1 erhaltenen Percarbamids unter ständigem Durchmischen erwärmt, zwischen 100° bis 120°C beginnt ein Sinterungs-Prozeß, der zur weitgehenden Verflüssigung und Homogenisierung des Produktes führt. Nach Kristallisation erhält man 10g einer Harnstoff-Einschlußverbindung mit einem H₂O₂-Gehalt von 15 % und einem Tensid-Gehalt von 5 %.

### Beispiel 5

Man verfährt mit dem nach Beispiel 1 hergestellten Carbamid nach der Arbeitsweise des Beispiels 4, wobei vorgefertigte Harnstoff-Tensid-Einschlußverbindungen - hergestellt nach der Arbeitsweise des Beispiels 2 - verwendet werden.

### Beispiel 6

Man erwärmt unter ständiger Durchmischung ein Gemisch von 10g des in Beispiel 1 erhaltenen Percarbamids mit 1g Hexadecansäuresorbitanester, 8g Harnstoff und 1g Allantoin. Man erhält nach dem Abkühlen 20g einer 4-Komponenten-Einschlußverbindung mit 15 % Wasserstoffperoxidgehalt.

### Beispiel 7

### Methodik

Die bakteriostatische Wirksamkeit der erfindungsgemäßen Einschlußverbindungen wurde im Plattenverdünnungstest nach Weuffen, Martin und Schade (Pharmazie 18 (1963) 420-426) bestimmt.

### Ergebnis:

Die erfindungsgemäßen Addukte zeigen sowohl gegen grampositive als auch gegen gramnegative Keime eine ausreichende Wirksamkeit.

### Beispiel 8

Konservierung einer mit Streptococcus faecium ATCC 6057 kontaminierten Nährlösung.

Methodik: Der Stamm wird auf Blutagar-Platten 24 h bei 37°C bebrütet. Nach mindestens 3 Subkulturen wird eine Streptococfaecium-Nährbouillon-Kultur hergestellt. 50 ml einer Lösung der erfindungsgemäßen Substanzen in abgestuften Konzentrationen beziehungsweise von H₂O₂ in Nährbouillon werden mit 0,1 ml dieser Kultur kontaminiert und 24 h, 48 h und 96 h bei Raumtemperatur aufbewahrt. Danach wird ein Allquot mit Katalase zur H₂O₂-Inaktivierung versetzt und auf festen Nährboden ausgespatelt und bebrütet. Das Wachstum wird nach 24 h, 48 h und 72 h beurteilt.

Ergebnis: Bei einer Konzentration von 0,25 % der Einschlußverbindungen wurde trotz massiver Kontamination und optimalen Wachstumsbedingungen kein Keimwachstum beobachtet. Bei einer Konzentration der Einschlußverbindungen von 0,00625 % bewirken diese noch eine weitgehende Hemmung des Bakterienwachstums, aber keine vollständige Inaktivierung. Eine 0,00625 %ige H₂O₂-Lösung bewirkt keine Hemmung des Bakterienwachstums.

### Beispiel 9

Ermittlung der Strahlenresistenz von Polioviren und der Beeinflussung durch Wasserstoffperoxid und die erfindungsgemäßen Verbindungen.

### Methodik:

Die Prüfung erfolgte nach v. Woedtke et al (1994)

### Ergebnis:

Die Inaktivierung von Polioviren durch Gammastrahlung ist in starkem Maße dosisabhängig. Es ist eine Mindestdosis von 20 kGy erforderlich. Durch Wasserstoffperoxid kann die erforderliche Strahlendosis stark herabgesetzt werden. Bei einem Zusatz der erfindungsgemäßen Einschlußverbindungen in einer Konzentration von 0,5 % waren alle Proben steril.

### Beispiel 10

### Sterilisation eines enzymatischen Glucosesensors:

Der Biosensor wird in eine 0,6 % bis 1 %ige Lösung der erfindungsgemäßen Substanzen oder in eine 0,5 %ige Lösung von H₂O₂ gebracht. die Konzentration der Lösung richtet sich nach der zu erwartenden maximal möglichen Kontamination. Die Lösung muß mindestens 70 h einwirken. Während dieses Zeitraums erfolgt eine Bestrahlung mit Gammastrahlen mit einer Strahlendosis in der Größenordnung von 6,2-6,5 kGy. Bis zur Anwendung lagern die Meßelektroden in der Lösung der erfindungsgemäßen Einschlußverbindungen oder in H₂O₂. Die Funktion des bioaktiven Elementes wird durch eine auf diese Art vorgenommene Sterilisation nicht meßbar beeinflußt.

### Beispiel 11:

Vergleich von toxischen Nebenwirkungen der erfindungsgemäßen Formulierungen im Vergleich zu Wasserstoffperoxid in der FL-Zellkultur (menschliche Amnionzellen).

### Methodik:

2 h nach der Zelleinsaat werden die Zellen, nachdem sie als einschichtige Monolayer vorliegen, nach Medienwechsel für 24 h mit der Prüfsubstanz exponiert, danach abgelöst und die Zellzahl in % zur Kontrolle mit dem Zellzähler Universal 870001 ermittelt (Kramer et al, Hyg. Med. 18 (1993), 9-16)

### Ergebnis:

Durch 0,001 % H₂O₂ werden etwa 25 % der Zellen gehemmt. Bei Einwirkung von 0,001 %igen Lösungen der erfindungsgemäßen Einschlußverbindungen werden die Zellen entweder gar nicht (EO 185/4, 185/6 und 185/8) oder sehr schwach gehemmt (EO 185/9 und EO 185/11).

## Patentansprüche

1. Verfahren zur Herstellung von antimikrobiellen Verbindungen auf Basis von Percarbamid-Einschlußverbindungen, dadurch gekennzeichnet, daß man die Percarbamid-Einschlußverbindungen und ein physiologisch tolerierbares Tensid direkt miteinander bei 50° bis 130°C zu einer Mehrkomponenten-Einschlußverbindung umsetzt.

2. Verfahren nach Anspruch 1, dadurch gekennzeichnet, daß man das Vermischen bei 100° bis 130° durchführt.

3. Verfahren nach Anspruch 1 und/oder 2, dadurch gekennzeichnet, daß man das Tensid in Form seiner Einschlußverbindung in Harnstoff einsetzt.

4. Verfahren nach einem der Ansprüche 1 bis 3, dadurch gekennzeichnet, daß ein oder mehrere weitere Wirkstoffe zugesetzt werden.

5. Verfahren nach Anspruch 4, dadurch gekennzeichnet, daß der weitere Wirkstoff in Form seiner Einschlußverbindung in Harnstoff zugesetzt wird.

6. Verfahren nach Anspruch 4, dadurch gekennzeichnet, daß die weiteren Wirkstoffe als Mehrkomponenten-Einschlußverbindung in Harnstoff zugesetzt werden.

7. Verfahren nach einem der Ansprüche 1 bis 6, dadurch gekennzeichnet, daß wasserfreie Einschlußverbindungen eingesetzt werden.

8. Verfahren nach einem der Ansprüche 1 bis 7, dadurch gekennzeichnet, daß man als Tensid einen Hexadecansäuresorbitanester einsetzt.

9. Verfahren nach einem der Ansprüche 1 bis 7, dadurch gekennzeichnet, daß man ein Tensidgemisch aus 66 % Tetradecansäuremonoglycerid und 34 % Hexadecansäurediethanolamid einsetzt.

10. Verwendung der nach den Ansprüchen 1 bis 9 erzeugten antimikrobiellen Verbindungen zur Konservierung von pharmazeutischen und kosmetischen Zubereitungen.

11. Verwendung nach Anspruch 10, dadurch gekennzeichnet, daß der Zubereitung des weiteren hautfreundliche oder haut pflegende Wirkstoffe zugesetzt sind.

12. Verwendung nach Anspruch 11, dadurch gekennzeichnet, daß der Zubereitung Allantoin oder Panthenol zugesetzt ist.

13. Verwendung der nach den Ansprüchen 1 bis 9 erzeugten antimikrobiellen Verbindungen zur Behandlung von empfindlichen Materialien.

## Claims

1. Method for preparing antimicrobial compounds based on percarbamide inclusion compounds, characterised in that the percarbamide inclusion compounds and a biocompatible tenside are reacted directly together at 50° to 130°C to produce a multi-component inclusion compound.

2. Method according to claim 1, characterised in that mixing is carried out at 100° to 130°.

3. Method according to claim 1 and/or 2, characterised in that the tenside is employed in the form of its inclusion compound in urea.

4. Method according to any of claims 1 to 3, characterised by the addition of one or more additional active agents.

5. Method according to claim 4, characterised in that the additional active agent is added in the form of its inclusion compound in urea.

6. Method according to claim 4, characterised in that the additional active agents are added as a multi-component inclusion compound in urea.

7. Method according to any of claims 1 to 6, characterised in that anhydrous inclusion compounds are employed.

8. Method according to any of claims 1 to 7, characterised in that the tenside employed is a hexadecanoic acid sorbitan ester.

9. Method according to any of claims 1 to 7, characterised by the use of a tenside mixture comprising 66% tetradecanoic acid monoglyceride and 34% hexadecanoic acid diethanol amide.

10. Use of the antimicrobial compounds produced according to claims 1 to 9 for preserving pharmaceutical and cosmetic preparations.

11. Use according to claim 10, characterised in that active agents that do not irritate the skin, or that nourish the skin, are further added to the preparation.

12. Use according to claim 11, characterised in that allantoin or panthenol is added to the preparation.

13. Use of the antimicrobial compounds produced according to claims 1 to 9 for treating delicate materials.

## Revendications

1. Procédé de préparation de composés antimicrobiens, à base de composés d'inclusion dans du percarbamide, caractérisé en ce qu'on fait réagir directement l'un avec l'autre les composés d'inclusion dans du percarbamide et un tensioactif acceptable d'un point de vue physiologique, à une température de 50 à 130°C, pour obtenir un composé d'inclusion multicomposants.

2. Procédé selon la revendication 1, caractérisé en ce que le mélange est mis en oeuvre à une température de 100 à 130°C.

3. Procédé selon la revendication 1 et/ou 2, caractérisé en ce qu'on utilise le tensioactif sous forme de son composé d'inclusion dans de l'urée.

4. Procédé selon l'une des revendications 1 à 3, caractérisé en ce qu'on ajoute une ou plusieurs autres matières actives.

5. Procédé selon la revendication 4, caractérisé en ce que l'autre matière active est ajoutée sous forme de son composé d'inclusion dans de l'urée.

6. Procédé selon la revendication 4, caractérisé en ce que les autres matières actives sont ajoutées sous forme d'un composé d'inclusion multicomposants dans de l'urée.

7. Procédé selon l'une des revendications 1 à 6, caractérisé en ce qu'on utilise des composés d'inclusion anhydres.

8. Procédé selon l'une des revendications 1 à 7, caractérisé en ce qu'on utilise en tant que tensioactif un ester de sorbitan de l'acide hexadécanoïque.

9. Procédé selon l'une des revendications 1 à 7, caractérisé en ce qu'on utilise un mélange de tensioactifs constitué de 66 % du monoglycéride de l'acide tétradécanoïque et de 34 % du diéthanolamide de l'acide hexadécanoïque.

10. Utilisation des composés antimicrobiens produits selon les revendications 1 à 9 pour la conservation de préparations pharmaceutiques et cosmétiques.

11. Utilisation selon la revendication 10, caractérisée en ce qu'en outre des matières actives, tolérées par la peau ou destinées aux soins de la peau, sont ajoutées à la préparation.

12. Utilisation selon la revendication 11, caractérisée en ce que de l'allantoïne ou du panthénol est ajouté à la préparation.

13. Utilisation des composés antimicrobiens produits selon les revendications 1 à 9, pour traiter des matériaux sensibles.
